(19) Europäisches Patentamt
European Patent Office
Office européen des brevets

(11) Veröffentlichungsnummer : **0 007 466**
**B1**

(12) # EUROPÄISCHE PATENTSCHRIFT

(45) Veröffentlichungstag der Patentschrift : **15.04.81**

(51) Int. Cl.³ : **C 07 F 9/40**, A 01 N 57/24

(21) Anmeldenummer : **79102207.2**

(22) Anmeldetag : **02.07.79**

(54) **2-Cyclopropyl-pyrimidin(4)yl-thionophosphonsäureester, Verfahren zu ihrer Herstellung, ihre Verwendung als Insektizide und Akarizide, diese enthaltende Schädlingsbekämpfungsmittel und Verfahren zu deren Herstellung.**

(30) Priorität : **15.07.78 DE 2831165**

(43) Veröffentlichungstag der Anmeldung : **06.02.80 (Patentblatt 80/03)**

(45) Bekanntmachung des Hinweises auf die Patenterteilung : **15.04.81 Patentblatt 81/15**

(84) Benannte Vertragsstaaten : **AT BE CH DE FR GB IT NL SE**

(56) Entgegenhaltungen :
**AT - B - 326 147**
**AT - B - 333 306**
**AT - B - 350 327**
**DE - B - 1 193 055**
**DE - C - 1 140 580**
**US - A - 3 216 894**
**US - A - 3 932 631**
**US - A - 4 115 542**

(73) Patentinhaber : **BAYER AG**
**Zentralbereich Patente, Marken und Lizenzen**
**D-5090 Leverkusen 1, Bayerwerk (DE)**

(72) Erfinder : **Maurer, Fritz, Dr.**
**Roeberstrasse 8**
**D-5600 Wuppertal 1 (DE)**
Erfinder : **Schröder, Rolf, Dr.**
**Pahlkestrasse 59**
**D-5600 Wuppertal 1 (DE)**
Erfinder : **Hammann, Ingeborg, Dr.**
**Belfortstrasse 9**
**D-5000 Köln 1 (DE)**
Erfinder : **Behrenz, Wolfgang, Dr.**
**Untergründemich 14**
**D-5063 Overath-Steinenbrück (DE)**
Erfinder : **Stendel, Wilhelm, Dr.**
**In den Birken 55**
**D-5600 Wuppertal 1 (DE)**

**EP 0 007 466 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

2-Cyclopropyl-pyrimidin(4)yl-thionophosphonsäureester, Verfahren zu ihrer Herstellung, ihre Verwendung als Insektizide und Akarizide, diese enthaltende Schädlingsbekämpfungsmittel und Verfahren zu deren Herstellung

Die Erfindung betrifft neue 2-Cyclopropyl-pyrimidin(4)yl-thionophosphonsäureester, ein Verfahren zu ihrer Herstellung sowie ihre Verwendung als Insektizide und Akarizide.

Es ist bekannt, daß 0-Alkyl-0-(2,6-dialkyl-pyrimidin(4)-yl)-thiono-phosphonsäureester, wie z.B. 0-Methyl-0-(2,6-dimethyl-pyrimidin(4)yl)-methan-thionophosphonsäureester, 0-Äthyl-0-(2,6-dimethyl-pyrimidin(4)yl-methan-thiono-phosphonsäureester und 0-Methyl-0-(2,6-dimethyl-pyrimidin(4)yl)-äthan-thionophosphonsäureester (vergleiche US-PS 3 216 894) und 0,0-Dialkyl-0-(2-cyclopropyl-6-methyl-pyrimidin(4)-thionophosphorsäureester, wie z.B. 0,0-Diäthyl-0-(2-cyclopropyl-6-methyl-pyrimidin(4)yl)-thionophosphorsäureester (vergleiche US-PS 4,012,506) insektizid und akarizid wirksam sind. Die Wirkung dieser Verbindungen ist jedoch, insbesondere bei niedrigen Aufwandmengen und Konzentrationen, nicht immer zufriedenstellend.

Es wurden nun die neuen 2-Cyclopropyl-pyrimidin(4)yl-thionophosphonsäureester der Formel

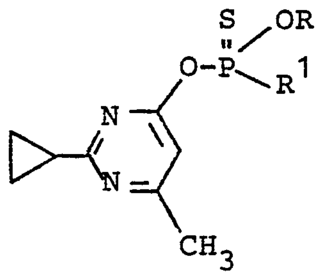
(I)

in welcher
R für Alkyl mit 1 bis 5 Kohlonstoffatomen steht und
$R^1$ für Alkyl mit 1 bis 5 Kohlonstoffatomen oder Phenyl steht,
gefunden.

Die neuen Wirkstoffe zeichnen sich durch ihre hohe Wirksamkeit bei der Bekämpfung von Schädlingen, insbesondere durch hohe insektizide und akarizide Wirksamkeit aus.

Weiter wurde gefunden, daß man die neuen 2-Cyclopropyl-pyrimidin(4)yl-thionophosphonsäureester der Formel (I) erhält, wenn man Thionophosphonsäureesterhalogenide der Formel

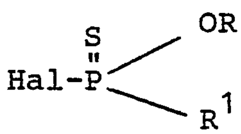
(II)

in welcher
R und $R^1$ die oben angegebene Bedeutung haben und
Hal für Chlor oder Brom steht,
mit 2-Cyclopropyl-6-methyl-4-hydroxy-pyrimidin der Formel

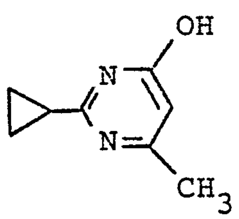
(III)

gegebenenfalls in Gegenwart eines Säureakzeptors und gegebenenfalls in Gegenwart eines Verdünnungsmittels umsetzt.

Überraschenderweise zeigen die erfindungsgemäßen 2-Cyclopropyl-pyrimidin(4)yl-thionophosphonsäureester eine bessere Wirksamkeit bei der Schädlingsbekämpfung, insbesondere durch eine bessere insektizide und akarizide wirkung als die entsprechenden aus dem Stand der Technik bekannten Verbindungen analoger Konstitution und gleicher Wirkungsrichtung. Die Produkte gemäß vorliegende Erfindung stellen somit eine wertvolle Bereicherung der Technik dar.

Verwendet man beispielsweise 0-iso-Propyl-n-propan-thionophosphonsäureesterchlorid und 2-Cyclopropyl-6-methyl-4-hydroxy-pyrimidin als Ausgangsstoffe, so kann die Reaktion durch folgendes Formelschema skizziert werden :

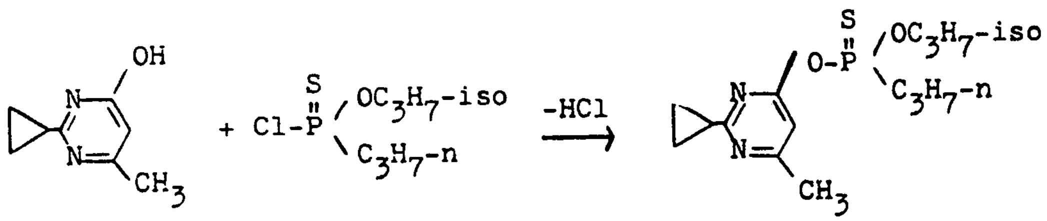

Die als Ausgangsstoffe zu verwendenden Thionophosphonsäureesterhalogenide sind durch Formel (II) definiert. Vorzugsweise stehen darin

R für geradkettiges oder verzweigtes Alkyl mit 1 bis 5, insbesondere mit 1 bis 3 Kohlenstoffatomen,

$R^1$ für geradkettiges oder verzweigtes Alkyl mit 1 bis 5, insbesondere mit 1 bis 3 Kohlenstoffatomen oder für Phenyl und

Hal für Chlor.

Die Ausgangsverbindungen der Formel (II) sind bekannt. Als Beispiele seien genannt:

0-Methyl-, 0-Äthyl-, 0-n-Propyl- und 0-iso-Propyl-methan-thionophosphonsäureesterchlorid, 0-Methyl-, 0-Äthyl-, 0-n-Propyl- und 0-iso-Propyl-äthan-thionophosphonsäureesterchlorid, 0-Methyl-, 0-Äthyl-, 0-n-Propyl- und 0-iso-Propyl-propan-thionophosphonsäureesterchlorid sowie 0-Methyl-, 0-Äthyl-, 0-n-Propyl- und 0-iso-Propyl-phenyl-thionophosphonsäureesterchlorid.

Das als zweite Reaktionskomponente einzusetzende 2-Cyclopropyl-6-methyl-4-hydroxy-pyrimidin ist bekannt (vergleiche US-PS 4 012 506).

Das Verfahren zur Herstellung der erfindungsgemäßen 2-Cyclopropyl-pyrimidin(4)yl-thionophosphonsäureester wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol, und 0-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Als Säureakzeptoren können alle üblichen Säurebindemittel Verwendung finden. Besonders bewährt haben sich Alkalicarbonate und -alkoholate, wie Natrium- und Kalium-carbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 100 °C, vorzugsweise bei 20 bis 80 °C. Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man ein organisches Lösungsmittel, z.B. Toluol, zu und arbeitet die organische Phase wie üblich durch Waschen, Trocknen, und Abdestillieren des Lösungsmittels auf.

Die neuen Verbindungen fallen in Form von Ölen an, die sich zum Teil nicht unzersetzt destillieren lassen, jedoch durch sogenanntes « Andestillieren », d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die erfindungsgemäßen 2-Cyclopropyl-pyrimidin(4)yl-thionphosphonsäure-ester zeichnen sich als Schädlingsbekämpfungsmittel, insbesondere durch eine hervorragende insektizide und akarizide Wirksamkeit gegenüber Pflanzen-, Hygiene- und Vorratsschädlingen aus. Sie besitzen eine gute Wirkung gegen saugende und fressende Insekten und Milben.

Aus diesem Grunde können die erfindungsgemäßen Verbindungen mit Erfolg im Pflanzenschutz sowie auf dem Hygiene-, Vorratsschutz- und Veterinärsektor als Schädlingsbekämpfungsmittel eingesetzt werden.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae,

Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp. Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

## Beispiel A

Plutella-Test

Lösungsmittel : 3 Gewichtsteile Aceton

Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Pluttella maculipennis) besetzt, solange die Blätter noch feucht sind.

Nach der gewünschten Zeit wird die Abtötung in % bestimmt. Dabei bedeutet 100 %, daß alle Raupen abgetötet wurden ; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik : 1, 2 und 3.

## Beispiel B

Tetranychus-Test (resistent)

Lösungsmittel : 3 Gewichtsteile Aceton

Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeiren Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden ; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik : 1, 3 und 4.

## Beispiel C

Mückenlarven-Test

Testtiere : Aedes aegypti, 4 Larven

Lösungsmittel : 99 Gewichtsteile Aceton

Emulgator : 1 Gewichtsteile Benzylhydroxydiphenyl-glykoläther

Zur Herstellung einer zweckmäßigen Wirkstoffzubereitung löst man 2 Gewichtsteile Wirkstoff in 1 000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wäßrigen Wirkstoffzubereitungen der gewünschten Konzentration in Gläser und setzt anschließend etwa 25 Mückenlarven in jedes Glas ein.

Nach 24 Stunden wird der Abtötungsgrad in % bestimmt. Dabei bedeutet 100 %, daß alle Larven abgetötet worden sind. 0 % bedeutet, daß überhaupt keine Larven abgetötet worden sind.

Bei diesem Test zeigen z.B. die folgenden Verbindungen der Herstellungsbeispiele überlegene Wirkung gegenüber dem Stand der Technik : 1, 2, 3 und 4.

## Beispiel D

Test mit parasitierenden adulten Rinderzecken (Boophilus microplus res.)

Lösungsmittel : Alkylarylpolyglykoläther

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (b. microplus res.) werden 1 Minute in die zu testende Wirkstoffzubereitung getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik : 1 und 3.

## Beispiel E

Test mit parasitierenden Fliegenlarven (Lucilia cuprina res.)

Emulgator : 80 Gewichtsteile Alkylarylpolyglykoläther

Zur Herstellung einer zweckmäßigen Wirkstoffzubereitung vermischt man 20 Gew.-Teile der betreffenden aktiven Substanz mit der angegebenen Menge des Emulgators und verdünnt das so erhaltene Gemisch mit Wasser auf die gewünschte Konzentration. Etwa 20 Fliegenlarven (Lucilia cuprina) werden in ein mit Wattestopfen entsprechender Größe beschicktes Teströhrchen gebracht, welches ca. 3 ml einer 20 % igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik : 1 und 3

## Herstellungsbeispiele

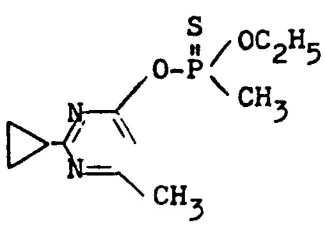

## Beispiel 1

Eine Mischung aus 15 g (0,1 Mol) 2-Cyclopropyl-4-hydroxy-6-methyl-pyrimidin, 20,7 g (0,15 Mol) Kaliumcarbonat, 15,9 g (0,1 Mol) 0-Äthyl-methanthionophosphonsäure-esterchlorid und 300 ml Acetonitril wird 4 Stunden bei 50 °C gerührt. Dann kühlt man den Ansatz auf Raumtemperatur ab und schüttelt ihn nach Zugabe von 400 ml Toluol zweimal mit je 300 ml Wasser aus. Die organische Phase wird abgetrennt, über Natriumsulfat getrocknet, im Vakuum vom Lösungsmittel befreit und der Rückstand andestilliert. Man erhält so 21,8 g (81 % der Theorie) 0-Äthyl-0-(2-cyclopropyl-6-methyl-pyrimidin(4)yl)-methanthionophosphonsäureester in Form eines gelben Öles mit dem Brechungsindex $n_D^{22}$ : 1,5367.

Analog Beispiel 1 können die folgenden Verbindungen der Formel

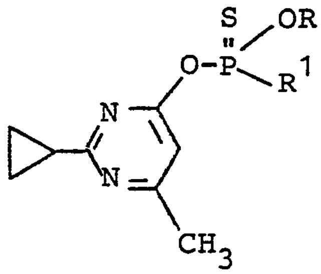

(I)

hergestellt werden :

| Beispiel Nr. | R | $R^1$ | Ausbeute (% der Theorie) | Brechungsindex: |
|---|---|---|---|---|
| 2 | $C_2H_5$ | 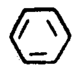 | 80 | $n_D^{22}$:1,5761 |
| 3 | $C_2H_5$ | $C_2H_5$ | 87 | $n_D^{22}$:1,5372 |
| 4 | $C_3H_7$-iso | $CH_3$ | 77 | $n_D^{22}$:1,5276 |
| 5 | $C_3H_7$-n | $C_2H_5$ | | |
| 6 | $CH_3$ | $CH_3$ | | |
| 7 | $CH_3$ | $C_2H_5$ | 86 | $n_D^{20}$:1,5404 |
| 8 | $CH_3$ | 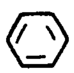 | | |

Das als Ausgangsmaterial einzusetzende 2-Cyclopropyl-4-hydroxy-6-methylpyrimidin kann zum Beispiel wie folgt hergestellt werden :

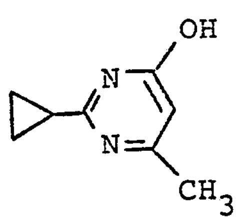

Zu einer Lösung von 70 g (1,3 Mol) Natriummethylat in 400 ml Methanol gibt man bei Raumtemperatur 78,3 g (0,65 Mol) Cyclopropylamidin-Hydrochlorid und anschließend 76,4 g (0,65 Mol) Acetessigsäuremethylester. Das Gemisch wird 18 Stunden bei Raumtemperatur gerührt, dann destilliert man das Lösungsmittel im Vakuum ab und löst den verbleibenden Rückstand in 400 ml Wasser. Durch Zugabe von konzentrierter Salzsäure wird auf pH 4 eingestellt und nach Abkühlen auf 5-10 °C saugt man das ausgefallene Produkt ab. Man erhält auf diese Weise 75 g (77 % der Theorie) 2-Cyclopropyl-4-hydroxy-6-methylpyrimidin in Form eines farblosen Pulvers mit dem Schmelzpunkt 187 °C.

**Ansprüche für die Vertragsstaaten :** BE, CH, DE, FR, GB, IT, NL, SE

1. 2-Cyclopropyl-pyrimidin(4)yl-thionophosphonsäureester der Formel

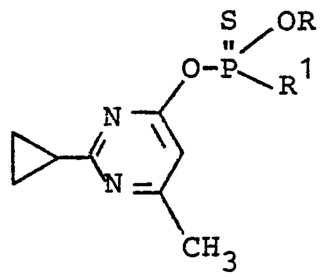

(I)

in der

R für Alkyl mit 1 bis 5 Kohlenstoffatomen steht und

$R^1$ Alkyl mit 1 bis 5 Kohlenstoffatomen oder Phenyl bedeutet

2. Verfahren zur Herstellung von 2-Cyclopropyl-pyrimidin(4)yl-thionophosphonsäureester der Formel (I), dadurch gekennzeichnet, daß man Thionophosphonsäureesterhalogenide der Formel

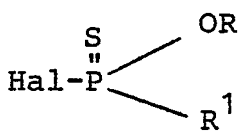

(II)

in der

R und $R^1$ die in Anspruch 1 angegebene Bedeutung haben und

Hal für Chlor oder Brom steht,

mit 2-Cyclopropyl-6-methyl-4-hydroxy-pyrimidin der Formel

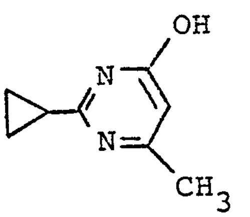

(III)

gegebenenfalls in Gegenwart eines Säureakzeptors und gegebenenfalls in Gegenwart eines Verdünnungsmittels umsetzt.

3. Schädlingsbekämpfungsmittel, gekennzeichnet durch einen Gehalt an Verbindungen gemäß Anspruch 1.

4. Insektizide und akarizide Mittel nach Anspruch 3, gekennzeichnet durch einen Gehalt an Verbindungen gemäß Anspruch 1.

5. Verwendung von Verbindungen gemäß Anspruch 1 zur Bekämpfung von Schädlingen, insbesondere von Insekten und Milben.

6. Verfahren zur Herstellung von Schädlingsbekämpfungsmitteln, insbesondere von insektiziden und akariziden Mitteln, dadurch gekennzeichnet, daß man Verbindungen gemäß Anspruch 1 mit Streckmitteln und/oder obenflächen-aktiven Mitteln mischt.

**Ansprüche für den Vertragsstaat :** AT

1. Verfahren zur Herstellung von 2-Cyclopropyl-pyrimidin(4)yl-thionophosphonsäureester der Formel I

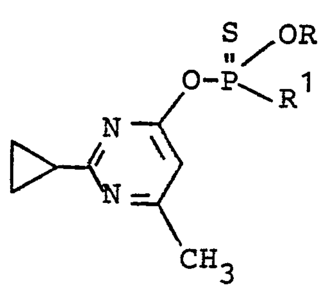

(I)

in der

R für Alkyl mit 1 bis 5 Kohlenstoffatomen steht und

$R^1$ Alkyl mit 1 bis 5 Kohlenstoffatomen oder Phenyl bedeutet, dadurch gekennzeichnet, daß man Thionophosphonsäureester-halogenide der Formel

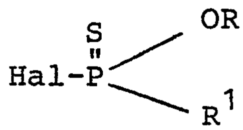

(II)

in der

R und $R^1$ die oben angegebene Bedeutung haben und

Hal für Chlor oder Brom steht,

mit 2-Cyclopropyl-6-methyl-4-hydroxy-pyrimidin der Formel

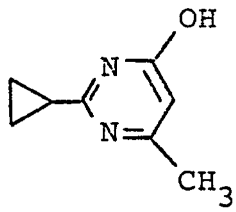

(III)

gegebenenfalls in Gegenwart eines Säureakzeptors und gegebenenfalls in Gegenwart eines Verdünnungsmittels umsetzt.

2. Schädlingsbekämpfungsmittel, gekennzeichnet durch einen Gehalt an Verbindungen der Formel I gemäß Anspruch 1.

3. Insektizide und akarizide Mittel nach Anspruch 2, gekennzeichnet durch einen Gehalt an Verbindungen der Formel I gemäß Anspruch 1.

4. Verwendung von Verbindungen der Formel I gemäß Anspruch 1 zur Bekämpfung von Schädlingen, insbesondere von Insekten und Milben.

5. Verfahren zur Herstellung von Schädlingsbekämpfungsmitteln, insbesondere von insektiziden und akariziden Mitteln, dadurch gekennzeichnet, daß man Verbindungen der Formel I gemäß Anspruch mit Streckmitteln und/oder obenflächenaktiven Mitteln mischt.

**Claims for the Contracting States :** BE, CH, DE, FR, GB, IT, NL, SE

1. 2-Cyclopropyl-pyrimidin-4-yl-thionophosphonic acid esters of the formula

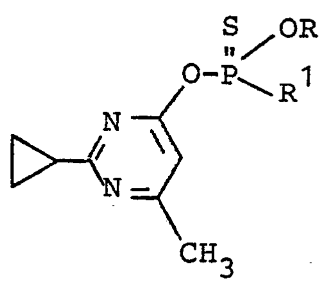

(I)

in which

R represents alkyl with 1 to 5 carbon atoms and

$R^1$ denotes alkyl with 1 to 5 carbon atoms or phenyl.

2. Process for the preparation of 2-cyclopropyl-pyrimidin-4-yl-thionophosphonic acid esters of the formula (I), characterised in that thionophosphonic acid ester halides of the formula

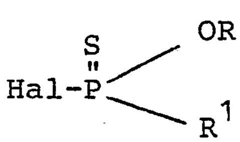

(II)

in which

R and $R^1$ have the meaning indicated in Claim 1 and Hal represents chlorine or bromine, are reacted

with 2-cyclopropyl-6-methyl-4-hydroxy-pyrimidine of the formula

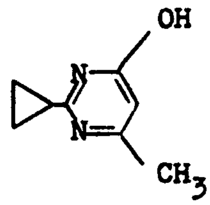

(III)

if appropriate in the presence of an acid acceptor and if appropriate in the presence of a diluent.

3. Agents for combating pests, characterised in that they contain compounds according to Claim 1.

4. Insecticidal and acaricidal agents according to Claim 3, characterised in that they contain compounds according to Claim 1.

5. Use of compounds according to Claim 1 for combating pests, in particular insects and mites.

6. Process for the preparation of agents for combating pests, in particular of insecticidal and acaricidal agents, characterised in that compounds according to Claim 1 are mixed with extenders and/or surface-active agents.

**Claims for the Contracting State :** AT

1. Process for the preparation of 2-cyclopropyl-pyrimidin-4-yl-thionophosphonic acid esters of the formula I

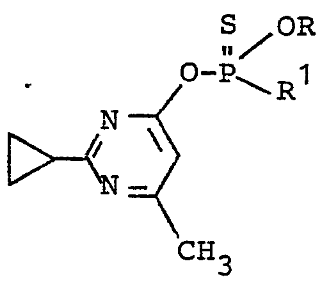

(I)

in which

R represents alkyl with 1 to 5 carbon atoms and

$R^1$ denotes alkyl with 1 to 5 carbon atoms or phenyl, characterised in that thionophosphonic acid ester halides of the formula

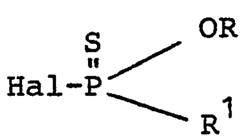

(II)

in which

R and $R^1$ have the meaning indicated above and Hal represents chlorine or bromine, are reacted with 2-cyclopropyl-6-methyl-4-hydroxy-pyrimidine of the formula

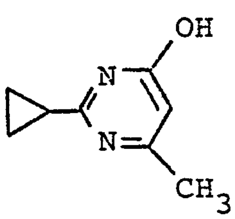

(III)

if appropriate in the presence of an acid acceptor and if appropriate in the presence of a diluent.

2. Agents for combating pests, characterised in that they contain compounds of the formula I according to Claim 1.

3. Insecticidal and acaricidal agents according to Claim 2, characterised in that they contain compounds of formula I according to Claim 1.

4. Use of compounds of formula I according to Claim 1 for combating pests, in particular insects and mites.

5. Process for the preparation of agents for combating pests, in particular of insecticidal and acaricidal agents, characterised in that compounds of formula I according to Claim 1 are mixed with extenders and/or surface-active agents.

**Revendications pour les Etats contractants :** BE, CH, DE, FR, GB, IT, NL, SE

1. Ester de 2-cyclopropyl-pyrimidin(4)yle d'acide thionophosphonique de formule :

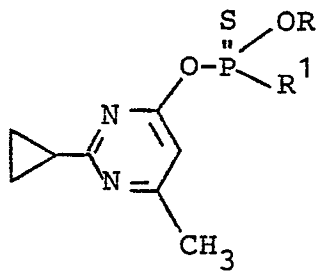

(I)

dans laquelle

R est un groupe alkyle ayant 1 à 5 atomes de carbone et

$R^1$ est un groupe alkyle ayant 1 à 5 atomes de carbone ou un groupe phényle.

2. Procédé de production d'esters de 2-cyclopropyl-pyrimidin-(4)-yle d'acide thionophosphonique de formule (I), caractérisé en ce qu'on fait réagir des halogénures d'esters d'acide thionophosphonique de formule :

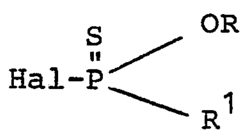

(II)

dans laquelle

R et $R^1$ ont la définition indiquée dans la revendication 1 et Hal est le chlore ou le brome, avec la 2-cyclopropyl-6-méthyl-4-hydroxy-pyrimidine de formule ;

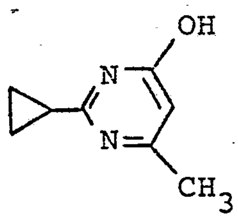

(III)

éventuellement en présence d'un accepteur d'acide et, le cas échéant, en présence d'un diluant.

3. Compositions pesticides, caractérisées en ce qu'elles contiennent des composés suivant la revendication 1.

4. Compositions insecticides et acaricides suivant la revendication 3, caractérisées en ce qu'elles contiennent des composés suivant la revendication 1.

5. Utilisation de composés suivant la revendication 1 pour la lutte contre des parasites, notamment des insectes et des acariens.

6. Procédé de production de compositions pesticides, notamment de compositions insecticides et acaricides, caractérisé en ce qu'on mélange des composés suivant la revendication 1 avec des diluants et/ou des agents tensio-actifs.

**Revendications pour l'Etat contractant :** AT

1. Procédé de production d'esters de 2-cyclopropyl-pyrimidin-(4)-yle d'acide thionophosphonique de formule I

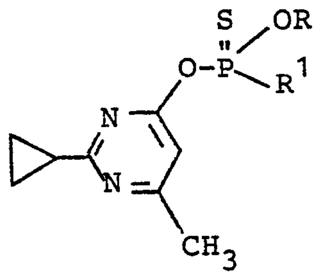

(I)

dans laquelle

R est un groupe alkyle ayant 1 à 5 atomes de carbone et

$R^1$ est un groupe alkyle ayant 1 à 5 atomes de carbone ou un groupe phényle caractérisé en ce qu'on fait réagir des halogénures d'esters d'acide thionophosphonique de formule :

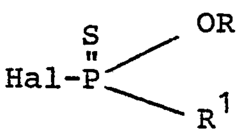

(II)

dans laquelle

R et $R^1$ ont la définition sus indiquée et Hal est le chlore ou le brome, avec la 2-cyclopropyl-6-méthyl-4-hydroxy-pyrimidine de formule :

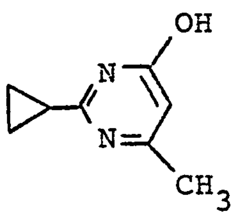

(III)

éventuellement en présence d'un accepteur d'acide et, le cas échéant, en présence d'un diluant.

2. Compositions pesticides, caractérisées en ce qu'elles contiennent des composés de formule I suivant la revendication 1.

3. Compositions insecticides et acaricides suivant la revendication 2, caractérisées en ce qu'elles contiennent des composés de formule I suivant la revendication 1.

4. Utilisation de composés de formule I suivant la revendication 1 pour la lutte contre des parasites, notamment des insectes et des acariens.

5. Procédé de production de compositions pesticides, notamment de compositions insecticides et acaricides, caractérisé en ce qu'on mélange des composés de formule I suivant la revendication 1 avec des diluants et/ou des agents tensio-actifs.